# EUROPEAN PATENT APPLICATION

(11) **EP 4 484 411 A1**
(43) Date of publication of application: **01.01.2025**
(21) Application number: 23760407.9
(22) Date of filing: 23.02.2023
(51) Int. Cl.: C07D 209/86, C07D 209/88, C07D 265/38, H10K 85/60, H10K 50/11

(54) **ORGANIC MOLECULE FOR OPTOELECTRONIC DEVICE**

(30) Priority: 23.02.2022 EP 22158231
(71) Applicant: Samsung Display Co., Ltd., Gyeonggi-do 17113 (KR)
(72) Inventor: DANZ, Michael, 76344 Eggenstein-Leopoldshafen (DE)
(74) Representative: Dr. Weitzel & Partner
(86) International application number: PCT/KR2023/002624
(87) International publication number: WO 2023/163533

(57) **Abstract**

The present disclosure relates to a pure organic molecule (free of a metal center) and use thereof as an emitter or absorber in an optoelectronic device. According to the present disclosure, the pure organic molecule includes a structure of Formula A. 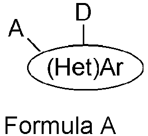 wherein
(Het)Ar = a conjugated organic group selected from the group consisting of unsubstituted and substituted aromatics, unsubstituted and substituted heteroaromatics, and conjugated double bonds fixed against cis-trans isomerization,
D = a chemically bonded donor group having electron donating properties,
A = a chemically bonded acceptor group having electron accepting properties, and
A and D are bonded to adjacent atoms of (Het)Ar.

## Description

### Technical Field

The present disclosure relates to a pure organic molecule (dye) of Formula A, free of a metal center, and use thereof as an emitter in organic light-emitting diodes (OLEDs) and other optoelectronic components.

### Background Art

Recently, technologies based on organic light-emitting diodes (OLEDs) have settled in the field of display technology, and the first commercial products based on these have emerged. In addition to screen technology, OLEDs are also suitable for flat lighting technology.

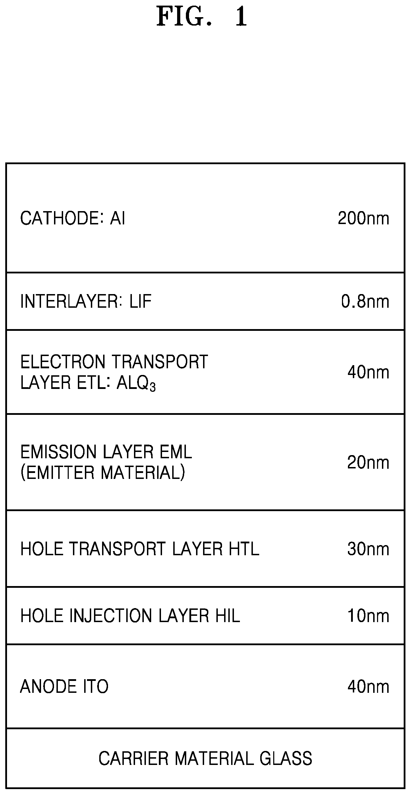

These devices are generally implemented as layered structures mainly consisting of organic materials. For better understanding, an example of a simplified structure is illustrated in FIG. 1. The key element of such a device is an emission layer in which a light-emitting molecule is generally embedded in a matrix. In the emission layer, negative charge carriers (electrons) and positive charge carriers (holes) meet and recombine to form so-called excitons (=excited state). Energy contained in the excitons can be emitted in the form of light by the corresponding emitter, which is referred to as electroluminescence. An overview of the functions of OLEDs is described in, for example, Top. Curr. Chem., 2004, 241, 1, H. Yersin, and "Highly Efficient OLEDs with Phosphorescent Materials," H Yersin; Wiley-VCH, Weinheim, Germany, 2018.

### Disclosure

### Technical Problem

Since the first report related to OLEDs (Tang et al. Appl. Phys. Lett. 1987, 51, 913), the above technology has continued to be developed, especially in the field of emitter materials. The first materials based on pure organic molecules can convert up to 25% of excitons into light according to spin statistics, but when phosphorescent compounds are used, this fundamental problem can be avoided. Thus, theoretically, all excitons can be converted into light. However, these materials have drawbacks, particularly in terms of cost and stability of materials used.

For triplet emitters suitable for triplet harvesting, transition metal complexes in which a metal is selected from the third-row transition metals are generally used. These are mainly very expensive precious metals such as iridium, platinum, or gold. (With regard to this, see the following: Top. Curr. Chem., 2004, 241, 1, H. Yersin, and Phys. Rev. B 1999, 60, 14422, M. A. Baldo, D. F. O'Brien, M. E. Thompson, S. R. Forrest).

### Technical Solution

Surprisingly, the above-described drawbacks can be considerably improved or solved by the present disclosure which uses an organic molecule (dye, emitter molecule) of Formula A.

The organic molecule according to the present disclosure includes a central basic unit in the form of a π-system (pi system), i.e., an aromatic (e.g., a heteroaromatic or an aromatic) which is substituted with a functional group having electron donating properties (donor) and a functional group having electron withdrawing properties (acceptor). Both groups are located on adjacent atoms of the central basic unit, i.e., at ortho-position relative to each other. Due to the ortho positioning of these groups (donor and acceptor), these groups have to rotate with respect to each other and thus be orthogonal to each other, resulting in electronic separation from each other. This separation may be understood, for example, by quantum mechanical calculations of HOMO and LUMO. Therefore, these two frontier orbitals must not overlap or must exhibit only minimal overlap. The π-system of the central basic unit may be mainly part of HOMO or LUMO.

Other than these groups (donor and acceptor), no group electronically affects the π-system (central basic unit). Permitted additional substituents may be alkyl groups or other groups that, for example, have a solubility effect.

The present disclosure provides an organic molecule comprising or consisting of a structure of Formula A which can be used in optoelectronic devices.

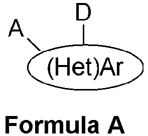

In formula A means:
(Het)Ar = a conjugated organic group selected from the group consisting of unsubstituted and substituted aromatics, unsubstituted and substituted heteroaromatics, and conjugated double bonds fixed against cis-trans isomerization,
D = a chemically bonded donor group having electron donating properties, and
A = a chemically bonded acceptor group having electron accepting properties, wherein A and D are bonded to directly adjacent atoms of the conjugated organic group (Het)Ar.

In an embodiment of the present disclosure, the organic molecule has a value ΔE(S₁-T₁) between the lowest excited singlet (S₁) state and a triplet (T₁) state therebelow of less than 5000 cm⁻¹, less than 3000 cm⁻¹, or less than 2000 cm⁻¹.

In an embodiment of the present disclosure, the organic molecule has an emission lifetime of at most 50 µs, at most 30 µs, or at most 20 µs.

In another embodiment, the organic molecule has both the value ΔE(S₁-T₁) presented herein and the emission lifetime presented herein.

Examples of donors include aromatics and heteroaromatics that inherently exhibit electron donating properties and/or are substituted with electron donating groups, and other donors directly attached to the central π-system (aromatic or heteroaromatic).

### Donors can be:

-O(-), -N(H)-alkyl group, -N-(alkyl group)₂-NH₂, -OH, -O-alkyl group, -NH(CO)-alkyl group, -O(CO)-alkyl group, -alkyl group, -phenyl group, and -(CH)=C-(alkyl group)₂.

Examples of acceptors include aromatics and heteroaromatics that inherently exhibit electron accepting properties and/or are substituted with electron accepting groups, and other donors directly attached to the central π-system (aromatic or heteroaromatic).

Acceptors can be:
-a halogen, -(CO)H, -(CO)-alkyl group, -(CO)O-alkyl group, -(CO)OH, -(CO)CI, - CF₃, -CN, -S(O)₂OH, - NH₃(+), -N(alkyl group)₃(+), and N(O)₂.

Optionally, the organic molecule may have at least one solubilizing group, for example, at least one alkyl group. The solubilizing group increases the solubility of the organic molecule in a given solvent.

In an embodiment, the substituent for increasing solubility (solubilizing group) may be selected from the group consisting of:
- branched or unbranched or cyclic long alkyl chains with a length of C₁ to C₃₀;
- branched or unbranched or cyclic alkoxy long chains with a length of C₁ to C₃₀;
- branched or unbranched or cyclic long perfluoroalkyl chains with a length of C₁ to C₃₀; and
- Short-chain polyethers with a chain length of 3 to 50 repeating units.

In an embodiment of the present disclosure, the organic molecule has a phenyl ring (Formula B) as the central π-system.

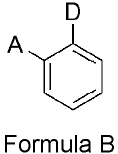

In another embodiment of the present disclosure, at least one of the two substituents (i.e. donor and/or acceptor) of the central basic unit includes a heteroatom X, and is bonded to the central basic unit (π-system) via the heteroatom.

The heteroatom X is independently selected from the group consisting of N, S, P, O, and Se.

In another embodiment of the present disclosure, the two substituents (i.e., donor and acceptor) may each independently be bonded to the central basic unit (π-system) via the heteroatom X selected from the group consisting of N, S, P, O, and Se.

In another embodiment of the present disclosure, the organic molecule has a structure of Formula C: 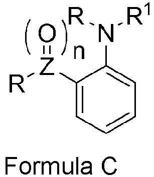 wherein
NRR¹ may be a donor (corresponding to D of Formula A or B),
R and R¹ may each independently be selected from
the group consisting of: hydrogen; a halogen; and a substituent bonded via a direct bond, or via oxygen- (-OR), nitrogen- (-NR₂), silicon atom- (-SiR₃), or a sulfur atom (-SR), and
the substituent may be selected from the group consisting of:
   alkyl groups (including branched or cyclic), heteroalkyl groups, aryl groups, heteroaryl groups, alkenyl groups, and alkynyl groups;
   alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
   other commonly known donor and acceptor groups such as amines, alcohol and ether groups, carbonyl groups, carboxylates and esters thereof, nitrile groups, and CF₃ groups.

Also, the residues R and R¹ may optionally lead to a fused ring system.
"Oxidized" heteroatom Z(=O)ₙ is an acceptor (corresponding to A of Formula A or B),
Z may be selected from the group consisting of S and P,
n may be 1 or 2, to which additional residue R is bonded,
the residue may each independently be selected from
the group consisting of: hydrogen; a halogen; and a substituent bonded via a direct bond, or via oxygen- (-OR), nitrogen- (-NR₂), silicon- (-SiR₃), or a sulfur atom (-SR), and
the substituent may be selected from the group consisting of:
   alkyl groups (including branched or cyclic), heteroalkyl groups, aryl groups, heteroaryl groups, alkenyl groups, and alkynyl groups;
   alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
   other commonly known donor and acceptor groups such as amines, alcohol and ether groups, carbonyl groups, carboxylates and esters thereof, nitrile groups, and CF₃ groups.

Also, the residues R may optionally lead to a fused ring system.

Examples of these acceptors include SO₂R, SO₂(OR), S(O)R, P(O)RR', P(O)(OR)(OR'), P(O)R(OR'), SeO₂R, and Se(O)R.

In another embodiment of the present disclosure, the organic molecule has a structure of Formula D: 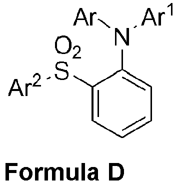 wherein
NArAr¹ may be a donor (corresponding to D of Formula A or B),
Ar and Ar¹ may each independently be aryl or heteroaryl to which additional residue R is optionally bonded,
the residue may each independently be selected from
the group consisting of: hydrogen; a halogen; and a substituent bonded via a direct bond, or via oxygen- (-OR), nitrogen- (-NR₂), silicon- (-SiR₃), or a sulfur atom (-SR), and
the substituent may be selected from the group consisting of:
   alkyl groups (including branched or cyclic), heteroalkyl groups, aryl groups, heteroaryl groups, alkenyl groups, and alkynyl groups;
   alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
   other commonly known donor and acceptor groups such as amines, alcohol and ether groups, carbonyl groups, carboxylates and esters thereof, nitrile groups, and CF₃ groups.

Also, the residue R may optionally lead to a fused ring system.
Ar and Ar¹ may also be fused with each other or linked to each other via unit E (e.g., carbazole or phenoxazine),
E is a direct bond, or
an organic bridge that is: a substituted or unsubstituted alkylene group, a substituted or unsubstituted alkenylene group, a substituted or unsubstituted alkynylene group, a substituted or unsubstituted arylene group, or a combination thereof;
-O-, -NR-, -SiR₂- -S-, -S(O)-, -S(O)₂-, an alkyl group interrupted by O (including branched or cyclic), a heteroalkyl group, an aryl group, a heteroaryl group, an alkenyl group, or a phenyl group;
or a substituted phenyl unit,
wherein R may each independently be selected from
the group consisting of: hydrogen; a halogen; and a substituent bonded via a direct bond, or via oxygen- (-OR), nitrogen- (-NR₂), silicon- (-SiR₃), or a sulfur atom (-SR), and
the substituent may be selected from the group consisting of:
   alkyl groups (including branched or cyclic), heteroalkyl groups, aryl groups, heteroaryl groups, alkenyl groups, and alkynyl groups;
   alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
   other commonly known donor and acceptor groups such as amines, alcohol and ether groups, carbonyl groups, carboxylates and esters thereof, nitrile groups, and CF₃ groups.

Also, the residue R may optionally lead to a fused ring system.
SO₂Ar² is an acceptor (corresponding to A of Formula A or B),
wherein Ar² may be aryl or heteroaryl to which additional residue R is optionally bonded,
the residue may each independently be selected from
the group consisting of: hydrogen; a halogen; and a substituent bonded via a direct bond, or via oxygen- (-OR), nitrogen- (-NR₂), silicon atom- (-SiR₃), or a sulfur atom (-SR), and
the substituent may be selected from the group consisting of:
   alkyl groups (including branched or cyclic), heteroalkyl groups, aryl groups, heteroaryl groups, alkenyl groups, and alkynyl groups;
   alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
   other commonly known donor and acceptor groups such as amines, alcohol and ether groups, carbonyl groups, carboxylates and esters thereof, nitrile groups, and CF₃ groups.

Also, the residue R may optionally lead to a fused ring system.

Ar may also be fused with other aryl and heteroaryl groups to form a larger aromatic system (e.g., phenyl, naphthyl, anthracenyl, tolyl, or the like).

In another embodiment of the present disclosure, the organic molecule has a structure of Formula E: 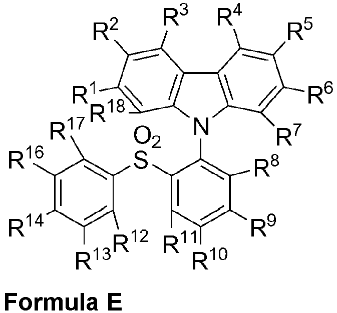 wherein
R¹ to R¹⁸ are each independently selected from the group consisting of:
hydrogen; a halogen; and a substituent bonded via a direct bond, or via oxygen-(-OR), nitrogen- (-NR₂), silicon- (-SiR₃), or a sulfur atom (-SR) (wherein the residue R is as defined above for R in Formula D), and
the substituent may be selected from the group consisting of:
   alkyl groups (including branched or cyclic), heteroalkyl groups, aryl groups, heteroaryl groups, alkenyl groups, and alkynyl groups;
   alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
   other commonly known donor and acceptor groups, for example, amines, alcohol and ether groups, carbonyl groups, carboxylates and esters thereof, nitrile groups, and CF₃ groups which are optionally further substituted and/or fused.

Also, the residues R¹ to R¹⁸ may optionally lead to a condensed ring system.

According to another aspect, the present disclosure relates to a method of preparing the above-described organic molecule. In an embodiment, the method comprises the step of substituting at least one of the three groups, i.e., (Het)Ar, A, and/or D (of Formula A or B) with at least one substituent for increasing solubility, wherein the substituent may be selected from the group consisting of:
- Long-chain, branched or unbranched or cyclic alkyl chains with a length of C₁ to C₃₀;
- Long-chain, branched or unbranched or cyclic alkoxy chains with a length of C₁ to C₃₀;
- Branched or unbranched or cyclic perfluoroalkyl chains with a length of C₁ to C₃₀; and
- Short-chain polyethers with a chain length of 3 to 50 repeating units.

According to another aspect, the present disclosure relates to use of the organic molecule as described above as an emitter or absorber in an optoelectronic device, and the optoelectronic device may be selected from the group consisting of:
- organic light-emitting devices (OLEDs),
- light-emitting electrochemical cells,
- OLED sensors, especially in gas and vapor sensors not hermetically externally shielded,
- organic solar cells,
- organic field-effect transistors,
- organic lasers, and
- down-conversion elements.

The proportion of the organic molecule in an emitter or an absorber is in a range of 1 % to 99 %. In an embodiment of the present disclosure, the proportion of the organic molecule in an emitter or an absorber may be 100 %.

In an embodiment, the proportion of the organic molecule as an emitter in optical light-emitting devices, particularly OLEDs is in a range of 5 % to 80 %.

According to another aspect, the present disclosure relates to an optoelectronic device including the organic molecule as described above. In particular, the device may be selected from the group consisting of organic light-emitting devices, organic diodes, organic solar cells, organic transistors, organic light-emitting diodes, light-emitting electrochemical cells, organic field-effect transistors, and organic lasers.

According to another aspect, the present disclosure relates to a method of producing an optoelectronic device using the organic molecule as described above.

Part of this method may be a step of applying the organic molecule according to the present disclosure to a carrier, and the application may optionally be performed by wet-chemical, by colloidal suspension or by sublimation.

According to another aspect, the present disclosure relates to a method of modifying the emission and/or absorption properties of an electronic device, the method including a step of introducing the organic molecule according to the present disclosure into a matrix material for conducting electrons or holes in an optoelectronic device.

According to another aspect, the present disclosure relates to use of the organic molecule as described above in an optoelectronic device, and relates to an optoelectronic device for converting (so-called down conversion) UV radiation or blue light into visible light, particularly green, yellow or red light.

### Description of Drawings

The drawings are as follows:
FIG. 1 schematically illustrates an example of the structure of an OLED (the device includes at least one anode, a cathode, and an emission layer, and the given thicknesses of layers are provided for illustrative purposes only).

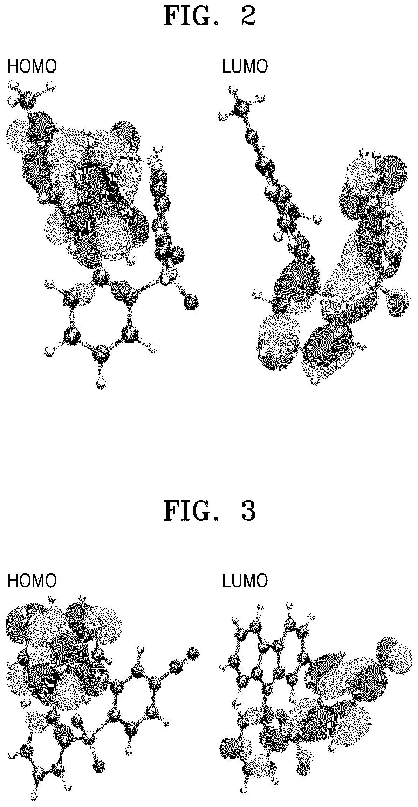

FIG. 2 is a HOMO/LUMO image of organic molecule 1.
FIG. 3 is a HOMO/LUMO image of organic molecule 2.

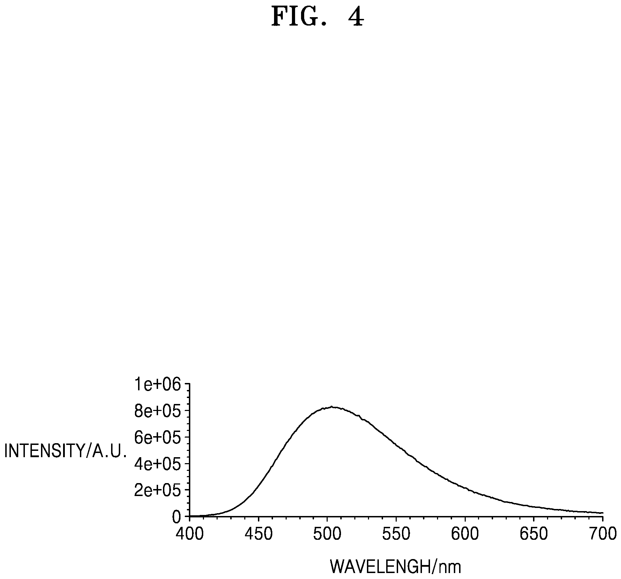

FIG. 4 illustrates the emission spectrum of organic molecule 1 dissolved in toluene (arbitrary units of intensity (a.u.) versus wavelength length (nm)).

### Mode for Invention

### Examples

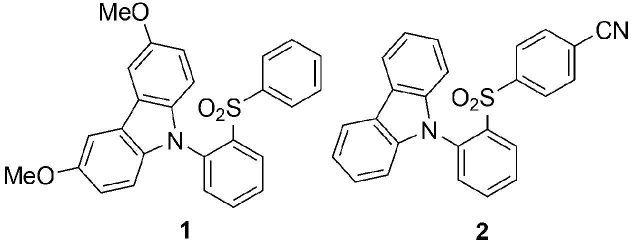

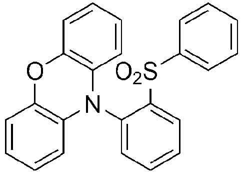

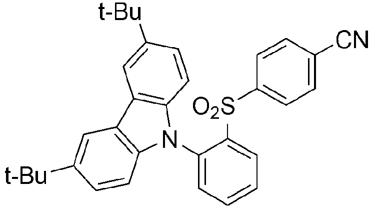

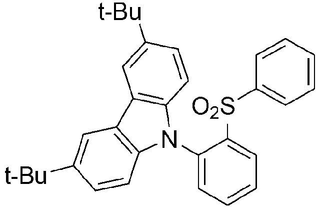

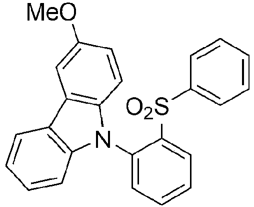

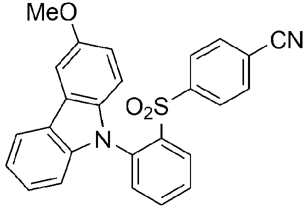

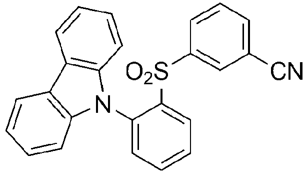

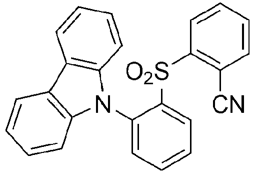

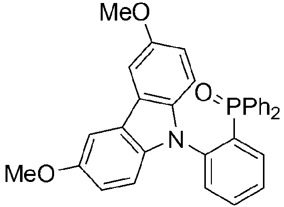

### Synthesis method

### Synthesis of 1

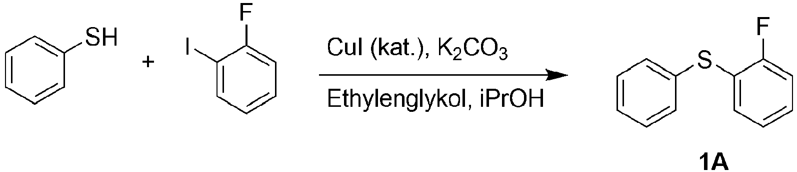

Copper(I) iodide (479 mg, 2.5 mmol) and potassium carbonate (13.8 g, 100 mmol) were placed in a 100 mL two-necked flask with a reflux condenser and evacuated for 5 minutes. Under atmosphere including nitrogen, 50 mL of 2-propanol, 1-fluoro-2-iodobenzene (12.2 g, 55 mmol), thiophenol (5.1 mL, 50 mmol), and ethylene glycol (5.6 mL, 100 mmol) were added. The mixture was stirred at 90 °C for 1 day. The reaction solution was filtered and the residue was washed with 100 mL of water and 100 mL of EtOAc. After phase separation, the aqueous phase was extracted again with 100 mL of EtOAC. The combined organic phase was washed with a saturated NaCl solution and dried over MgSO₄. The solvent was removed under reduced pressure on a rotary evaporator and the residue was filtered through a small amount of silica gel (eluent: CH₂Cl₂). The solvent was removed again under reduced pressure on a rotary evaporator. 9.8 g of diarylsulfide 1A was obtained as colorless oil that contained approximately 10 % 1-fluoro-2-iodobenzene but could react further without further purification.

¹H-NMR (500 MHz, CDCl₃): δ = 7.41-7.37 (m, 2H), 7.37-7.32 (m, 2H), 7.32-7.27 (m, 3H), 7.16-7.08 (m, 2H).

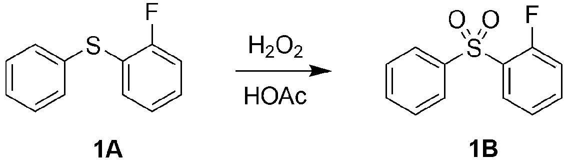

Diarylsulfide 1A (purity 90 %, 6.81 g, 30 mmol) was dissolved in 150 mL of glacial acetic acid and treated with 15 mL of H₂O₂ (30 % in water). The mixture was stirred at 100 °C for 2 hours, and then left at room temperature overnight. The reaction solution was poured into 800 mL of water, causing the product to precipitate as a white solid. The solid was filtered and washed with water. The resulting filtrate was dried under vacuum at 50 °C overnight. 5.8 g of diarylsulfone 1B was obtained as a white solid.

¹H-NMR (500 MHz, CDCl₃) δ = 8.11 (td, J = 7.5, 1.8 Hz, 1H), 8.05 - 7.98 (m, 2H), 7.65 - 7.55 (m, 2H), 7.53 (t, *J* = 7.8 Hz, 2H), 7.32 (t, *J* = 7.7 Hz, 1H), 7.14 - 7.07 (m, 1H).

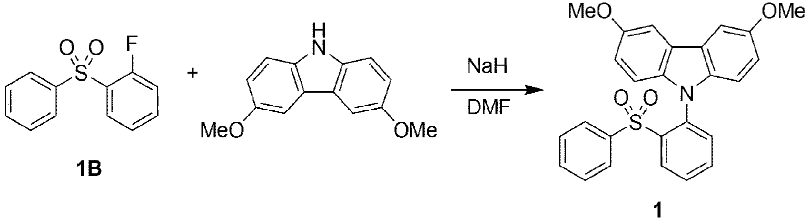

3,6-dimethoxycarbazole (696 mg, 3 mmol) was dissolved in 10 mL of dry dimethylformamide (DMF) and NaH (60 % in mineral oil, 240 mg, 6 mmol) was added. After stirring for 30 minutes, diarylsulfone 1B dissolved in 10 mL of DMF was added and the mixture was stirred at 100 °C for 2 hours. The reaction solution was poured into 200 mL of ice water. After being left overnight, the solid was filtered and washed with some water. The solid was purified by column chromatography (eluent: CH₂Cl₂/cyclohexane). 350 mg of product 1 was obtained, which could further be purified by recrystallization from EtOH.

¹H-NMR (500 MHz, CDCl₃) δ = 8.61 (dd, *J* = 7.8, 1.8 Hz, 1H), 7.74 (dtd, *J* = 18.7, 7.5, 1.6 Hz, 2H), 7.44 (d, *J* = 2.5 Hz, 2H), 7.13 (ddt, *J* = 5.5, 4.0, 2.2 Hz, 2H), 7.11 - 7.07 (m, 2H), 6.88 (t, *J* = 7.9 Hz, 2H), 6.76 (dd, *J* = 8.8, 2.5 Hz, 2H), 6.41 (d, *J* = 8.8 Hz, 2H), 3.92 (s, 6H).

¹³C-NMR (125 MHz, CDCl₃) δ = 154.3, 142.7, 139.4, 138.2, 137.1, 135.3, 132.8, 132.8, 130.0, 129.7, 128.4, 127.9, 123.9, 115.0, 111.6, 102.7, 56.3.

### Synthesis of 2

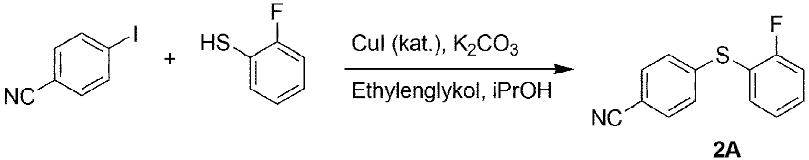

4-iodobenzonitrile (11.5 g, 500 mmol), copper(I) iodide (957 mg, 5 mmol), and potassium carbonate (13.8 g, 100 mmol) were placed in a 250 mL two-necked flask with a reflux condenser and evacuated for 5 minutes. Under nitrogen atmosphere, 100 mL of 2-propanol (iPrOH), 1-fluoro-2-thiobenzene (6.41 g, 50 mmol), and ethylene glycol (5.6 mL, 100 mmol) were added. The mixture was stirred at 90 °C for 1 day. The reaction solution was filtered and the residue was washed with 300 mL of EtOAc. The solvent was removed under reduced pressure on a rotary evaporator, and the residue was dissolved in 100 mL of EtOAc and 100 mL of water. The organic phase was washed once more with 100 mL water, followed by washing with 50 mL of a saturated NaCl solution and drying over MgSO₄. The solvent was removed under reduced pressure on a rotary evaporator and the residue was filtered through a small amount of silica gel (eluent: CH₂Cl₂). The solvent was removed again under reduced pressure on a rotary evaporator. 10.5 g of diarylsulfide 2A was obtained as a white solid, which contains about 5 % 4-iodobenzonitrile but can further be converted without further purification.

¹H-NMR (500 MHz, CDCl₃): δ = 7.57 - 7.43 (m, 4H), 7.25 - 7.19 (m, 2H), 7.19 - 7.15 (m, 2H).

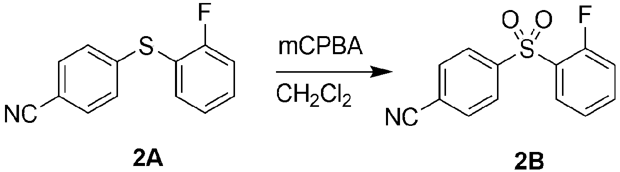

Diarylsulfide 2A (purity 95 %, 5.31 g, 22 mmol) was dissolved in 150 mL of CH₂Cl₂. Meta-chloroperbenzoic acid (mCPBA) (70 %, 16.3 g, 66 mmol) was added over approximately 10 minutes under ice cooling. Subsequently, the mixture was stirred at room temperature for 16 hours. The reaction solution was washed with 100 mL of a semiconcentrated Na₂CO₃ solution, and then washed with 50 mL of a saturated NaCl solution and dried over MgSO₄. The solvent was removed under reduced pressure on a rotary evaporator and the residue was filtered through a small amount of silica gel (eluent: CH₂Cl₂). The solvent was removed again under reduced pressure on a rotary evaporator. 5.2 g of diarylsulfone 2B was obtained as a light yellow solid.

1H-NMR (500 MHz, CDCl₃): δ = 8.17 - 8.08 (m, 3H), 7.85 - 7.81 (m, 2H), 7.64 (tdd, J = 7.4, 4.9, 1.8 Hz, 1H), 7.37 (t, J = 7.7 Hz, 1H), 7.18 -7.10 (m, 1H).

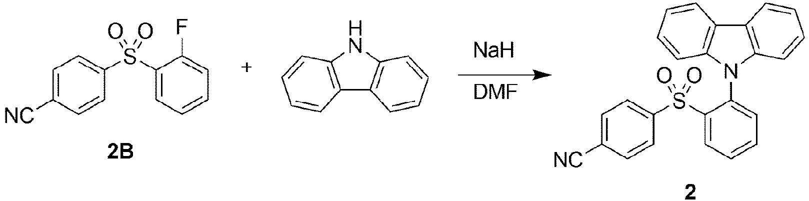

Carbazole (2.51 g, 15 mmol) was dissolved in 50 mL of dry DMF and NaH (60 % in mineral oil, 1.20 g, 30 mmol) was added. After stirring for 15 minutes, diarylsulfone 2B dissolved in 50 mL of DMF was added and the mixture was stirred at 100 °C for 2 hours. The reaction solution was poured onto 800 mL of ice. After being left overnight, the solid was filtered and washed with water. The solid was purified by column chromatography (eluent: CH₂Cl₂). 3.14 g of product 2 was obtained as a white solid.

¹H-NMR (500 MHz, CDCl₃): δ = 8.66 - 8.61 (m, 1H), 8.03 (d, *J* = 7.7 Hz, 2H), 7.89 - 7.80 (m, 2H), 7.30 - 7.23 (m, 5H), 7.19 (ddd, *J* = 8.2, 7.1, 1.2 Hz, 2H), 7.11 - 7.01 (m, 4H), 6.53 (d, *J =* 8.0 Hz, 2H).

¹³C-NMR (125 MHz, CDCl₃) δ = 142.8, 141.9, 141.8, 136.1, 136.0, 133.0, 132.0, 130.3, 130.2, 128.1, 126.1, 123.3, 120.8, 120.1, 117.1, 116.2, 110.4.

## Claims

1. An organic molecule having a structure of Formula A: 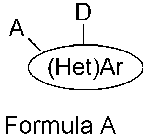 wherein
(Het)Ar = a conjugated organic group selected from the group consisting of unsubstituted and substituted aromatics, unsubstituted and substituted heteroaromatics, and conjugated double bonds fixed against cis-trans isomerization,
D = a chemically bonded donor group having electron donating properties, A = a chemically bonded acceptor group having electron accepting properties, and
A and D are bonded to directly adjacent atoms of the conjugated organic group (Het)Ar.

2. The organic molecule of claim 1, wherein the organic molecule has
a value ΔE(S₁-T₁) between a lowest excited singlet (S₁) state and a triplet (T₁) state below the lowest excited singlet (S₁) state of less than 5000 cm⁻¹ and/or
an emission lifetime of 50 µs or less.

3. The organic molecule of claim 1 or 2, wherein (Het)Ar, A, and/or D has at least one substituent increasing solubility of the organic molecule in an organic solvent, wherein the substituent increasing solubility is preferably selected from the group consisting of:
- branched or unbranched or cyclic long alkyl chains with a length of C₁ to C₃₀;
- branched or unbranched or cyclic long alkoxy chains with a length of C₁ to C₃₀;
- branched or unbranched or cyclic long perfluoroalkyl chains with a length of C₁ to C₃₀; and
- Short-chain polyethers with a chain length of 3 to 50 repeating units.

4. The organic molecule of claims 1 to 3, wherein (Het)Ar is phenyl.

5. The organic molecule of claims 1 to 4, wherein at least one of the two substituents A and/or D has a heteroatom X through which the substituent is covalently bonded to (Het)Ar.

6. The organic molecule of claim 5, wherein the heteroatom X is selected from the group consisting of N, S, P, O, and Se.

7. The organic molecule of claims 1 to 6, wherein the organic molecule has a structure of Formula C: 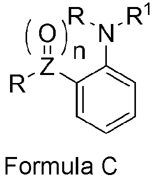 wherein
NRR¹ is a donor,
R and R¹ are each independently selected from
the group consisting of: hydrogen; a halogen; and a substituent bonded via a direct bond, or via oxygen- (-OR), nitrogen- (-NR₂), silicon- (-SiR₃), or a sulfur atom (-SR),
the substituent is selected from the group consisting of:
alkyl groups (including branched or cyclic), heteroalkyl groups, aryl groups, heteroaryl groups, alkenyl groups, and alkynyl groups;
alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
other commonly known donor and acceptor groups such as amines, alcohol and ether groups, carbonyl groups, carboxylates and esters thereof, nitrile groups, and CF₃ groups,
the residues R and R¹ optionally lead to a fused ring system,
heteroatom Z(=O)ₙ is an acceptor (corresponding to A of Formula A or B), Z is selected from the group consisting of S and P,
n is 1 or 2, to which additional residue R is bonded,
the residues are each independently selected from
the group consisting of: hydrogen; halogens; and a substituent bonded via a direct bond, or via oxygen- (-OR), nitrogen- (-NR₂), silicon- (-SiR₃), or a sulfur atom (-SR),
the substituent is selected from the group consisting of:
alkyl groups (including branched or cyclic), heteroalkyl groups, aryl groups, heteroaryl groups, alkenyl groups, and alkynyl groups;
alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
other commonly known donor and acceptor groups such as amines, alcohol and ether groups, carbonyl groups, carboxylates and esters thereof, nitrile groups, and CF₃ groups, and
the residues R optionally lead to a fused ring system.

8. The organic molecule of claims 1 to 7, wherein the organic molecule has a structure of Formula D: 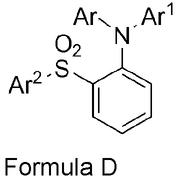 wherein
NArAr¹ is a donor (corresponding to D of Formula A or B),
Ar and Ar¹ are each independently aryl or heteroaryl to which an additional residue R is optionally bonded,
the residue R is selected from
the group consisting of: hydrogen; halogens; and a substituent bonded via a direct bond, or via oxygen- (-OR), nitrogen- (-NR₂), silicon- (-SiR₃), or a sulfur atom (-SR),
the substituent is selected from the group consisting of:
alkyl groups (branched or cyclic), heteroalkyl groups, aryl groups, heteroaryl groups, alkenyl groups, and alkynyl groups;
alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
other commonly known donor and acceptor groups such as amines, alcohol and ether groups, carbonyl groups, carboxylates and esters thereof, nitrile groups, and CF₃ groups,
the residues R optionally lead to a fused ring system,
Ar and Ar¹ are also fused with each other or linked to each other via unit E,
E is a direct bond, or
an organic bridge that is: a substituted or unsubstituted alkylene group, a substituted or unsubstituted alkenylene group, a substituted or unsubstituted alkynylene group, a substituted or unsubstituted arylene group, or a combination thereof;
-O-, -NR-, -SiR₂- -S-, -S(O)-, -S(O)₂-, an alkyl group interrupted by O (including branched or cyclic), a heteroalkyl group, an aryl group, a heteroaryl group, an alkenyl group, or a phenyl group;
or a substituted phenyl unit,
wherein each R is independently selected from
the group consisting of: hydrogen; halogens; and a substituent bonded via a direct bond, or via oxygen- (-OR), nitrogen- (-NR₂), silicon- (-SiR₃), or a sulfur atom (-SR),
the substituent is selected from the group consisting of:
alkyl groups (including branched or cyclic), heteroalkyl groups, aryl groups, heteroaryl groups, alkenyl groups, and alkynyl groups;
alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
other commonly known donor and acceptor groups such as amines, alcohol and ether groups, carbonyl groups, carboxylates and esters thereof, nitrile groups, and CF₃ groups,
the residues R optionally lead to a fused ring system,
SO₂Ar² is an acceptor (corresponding to A of Formula A or B),
wherein Ar² is aryl or heteroaryl to which an additional residue R is optionally bonded,
the residues are each independently selected from the group consisting of: hydrogen; halogens; and a substituent bonded via a direct bond, or via oxygen- (-OR), nitrogen- (-NR₂), silicon atom- (-SiR₃), or a sulfur atom (-SR),
the substituent is selected from the group consisting of:
alkyl groups (branched or cyclic), heteroalkyl groups, aryl groups, heteroaryl groups, alkenyl groups, and alkynyl groups;
alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
other commonly known donor and acceptor groups such as amines, alcohol and ether groups, carbonyl groups, carboxylates and esters thereof, nitrile groups, and CF₃ groups,
the residues R optionally lead to a fused ring system, and
Ar is optionally fused with other aryl groups and heteroaryl groups to form a larger aromatic system.

9. The organic molecule of claims 1 to 7, wherein the organic molecule has a structure of Formula E: 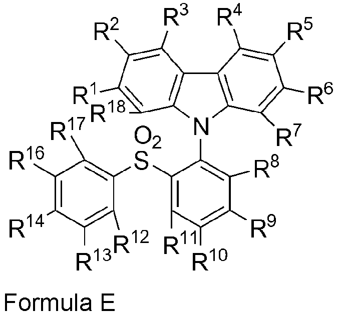 wherein
R¹ to R¹⁸ are each independently selected from the group consisting of: hydrogen; halogens; and a substituent bonded via a direct bond, or via oxygen- (-OR), nitrogen- (-NR₂), silicon- (-SiR₃), or a sulfur atom (-SR) (wherein the residue R is as defined above for R in Formula D),
the substituent is selected from the group consisting of:
alkyl groups (including branched or cyclic), heteroalkyl groups, aryl groups, heteroaryl groups, alkenyl groups, and alkynyl groups;
alkyl groups (including branched or cyclic), heteroalkyl groups, aryl groups, heteroaryl groups, and alkenyl groups, each substituted with a substituent such as halogens, deuterium, alkyl groups (including branched or cyclic), a heteroalkyl group, an aryl group, or a heteroaryl group; and
other commonly known donor and acceptor groups, for example, amines, alcohol and ether groups, carbonyl groups, carboxylates and esters thereof, nitrile groups, and CF₃ groups which are optionally further substituted and/or fused, and
the residues R¹ to R¹⁸ optionally lead to a condensed ring system.

10. A method of preparing the organic molecule according to claims 1 to 9.

11. The method of claim 10, further comprising substituting at least one of the three groups (Het)Ar, A and/or D with at least one substituent for increasing solubility, wherein the substituent is selected from the group consisting of:
- Long-chain, branched or unbranched or cyclic alkyl chains with a length of C₁ to C₃₀;
- Long-chain, branched or unbranched or cyclic alkoxy chains with a length of C₁ to C₃₀;
- Branched or unbranched or cyclic perfluoroalkyl chains with a length of C₁ to C₃₀; and
- Short-chain polyethers with a chain length of 3 to 50 repeating units.

12. Use of the organic molecule according to claims 1 to 9 as an emitter or absorber in an optoelectronic device.

13. The use of claim 12, wherein the optoelectronic device is selected from the group consisting of:
- organic light-emitting devices (OLEDs),
- light-emitting electrochemical cells,
- OLED sensors, especially in gas and vapor sensors not hermetically externally shielded,
- organic solar cells,
- organic field-effect transistors,
- organic lasers, and
- down-conversion elements.

14. The use of claim 12 or 13, wherein a proportion of the organic molecule in an emitter or an absorber is in a range of 1 % to 99 % or 100 %.

15. The use of claims 12 to 14, wherein a proportion of the organic molecule as an emitter in an optoelectronic device, particularly an OLED, is in a range of 5 % to 80 %.

16. An optoelectronic device comprising the organic molecule according to claims 1 to 9.

17. The optoelectronic device of claim 16, wherein the optoelectronic device is formed as a device selected from the group consisting of organic light-emitting devices, organic diodes, organic solar cells, organic transistors, organic light-emitting diodes, light-emitting electrochemical cells, organic field-effect transistors, and organic lasers.

18. A method of producing an optoelectronic device using the organic molecule according to claims 1 to 9.

19. The method of claim 18, wherein the method comprises applying the organic molecule according to claims 1 to 9 to a carrier, wherein the applying is optionally performed by wet-chemical, by colloidal suspension or by sublimation.

20. A method of modifying emission and/or absorption characteristics of an electronic device, the method comprising introducing the organic molecule according to claims 1 to 9 into a matrix material for conducting electrons or holes in an optoelectronic device.

21. Use of the organic molecule according to claims 1 to 9 particularly in an optoelectronic device for converting (down-converting) UV radiation or blue light into visible light, particularly green, yellow or red light.
